# EUROPEAN PATENT APPLICATION

(11) **EP 4 339 206 A1**
(43) Date of publication of application: **20.03.2024**
(21) Application number: 22906548.7
(22) Date of filing: 13.12.2022
(51) Int. Cl.: C07K 16/26, C12N 15/13

(54) **ANTIBODY AGAINST INHIBIN AND USE THEREOF**

(30) Priority: 15.12.2021 CN 202111539705
(71) Applicant: Ningbo Sansheng Biological Technology Co., Ltd., Ningbo, Zhejiang 315171 (CN)
(72) Inventor: ZHANG, Yong, Ningbo, Zhejiang 315171 (CN); ZHENG, Xingchang, Ningbo, Zhejiang 315171 (CN); WANG, Aike, Ningbo, Zhejiang 315171 (CN); JIN, Jiamin, Ningbo, Zhejiang 315171 (CN); YANG, Jiale, Ningbo, Zhejiang 315171 (CN); SUN, Di, Ningbo, Zhejiang 315171 (CN); CHEN, Yu, Ningbo, Zhejiang 315171 (CN); YANG, Junshuai, Ningbo, Zhejiang 315171 (CN); CHEN, Yong, Ningbo, Zhejiang 315171 (CN); CHEN, Lu, Ningbo, Zhejiang 315171 (CN); ZHOU, Sichao, Ningbo, Zhejiang 315171 (CN)
(74) Representative: Elzaburu S.L.P.
(86) International application number: PCT/CN2022/138630
(87) International publication number: WO 2023/109803

(57) **Abstract**

The present disclosure provides an anti-inhibin antibody and its applications. The antibody can have the inhibitory effect of inhibins on the secretion of endogenous follicle-stimulating hormone (FSH), promote the secretion of endogenous FSH and promote the development of animal follicles. The antiobidy can replace the use of FSH or pregnant mare serum Gonadotropin (PMSG) in animal reproduction, and can be used for promoting animal reproduction, estrus synchronization, conception and birth, embryo transferring, improving ovulation quality, promoting livestock reproduction, inducing estrus in female livestock.

## Description

### Technical Field

The present disclosure relates to the technical field of animal reproduction, and more particularly to an anti-inhibin antibody and its applications in assisting in animal reproduction.

### Background

Inhibin is a water-soluble protein hormone secreted by animal gonads and belongs to the transforming growth factor-β superfamily (TGF-β), which is named for its strong inhibitory effect on the production and secretion of pituitary follicle-stimulating hormone (FSH). Inhibin exists in the form of αβ heterodimers, and the β subunits are divided into five types: βA, βB, βC, βD, and βE. At present, inhibin A (αβA) and inhibin B (αβB) are more studied, both can inhibit the synthesis and secretion of FSH. Inhibin A is mainly secreted by dominant follicles and corpus luteum, and inhibin B is secreted by small and medium antral follicles. It has been reported in a large number of studies that neutralizing inhibin by active or passive immunizationand promoting the production and secretion of endogenous FSH can stimulate follicle development and increase the number of ovulation in female animals, indicating that both methods have the potential to regulate the reproductive performance of livestock. It is of great value to livestock production and economic animal reproduction.

At present, it has been reported in relevant patents and articles that, coupling partial peptides of α subunits of inhibin with some carrier proteins, or directly using recombinant α subunits of inhibin as an immunogen to actively immunize female animals, thereby relieving the inhibitory effect on endogenous FSH by inhibin. The ovulation number is significantly increased after immunization. However, since inhibin is an influencing factor for fertilized egg implantation, long-term antibody neutralization of inhibin induced by active immunization has negative effects on conception rate, which could possibly cause decrease of conception while ovulation rate increases.

At the same time, the effects of passive immunization against inhibin on the reproductive performance of female animals have also been extensively studied. The corresponding anti-inhibin serum (AIS) was prepared by immunizing animals with antigens made by coupling partial peptides of α subunits of inhibin with some carrier proteins, and then injecting AIS into female animals to neutralize endogenous inhibin through passive immunization. This approach significantly increased ovulation number in mice, goats, pigs, cattle, horses, and fish. According to the patent application (CN111134084A) of which the inventiors areby Pan Jianzhi, and the applicant is Zhejiang Academy of Agricultural Sciences, etc., AIS has also shown a better clinical effect than that of pregnant mare serum gonadotropin (PMSG) in a fixed-time artificial insemination of pigs, which is superior to PMSG in ovulation number, reducing an ovarian cyst incidence and improving the hormone secretion of replacement gilts. It can be seen that if AIS replaces PMSG in superovulation or a fixed-time artificial insemination, the reproductive performance of female animals will be further improved. However, AIS is a polyclonal antibody having complex components and large batch-to-batch differences, which hardly meet the basic principle of drug quality control. In addition, it needs to repeatedly inject immunogens into animals during each preparation time, causing a long preparation period. And finally it also needs to collect the blood of immunized animals, during the immunization process may lead to abnormal death of animals and is not in line with animal ethics.

A patent reference (WO1991010449A1)pubished in 1991 mentioned application of neutralizing antibodies using inhibin to stimulate superovulation in domesticated mammals, which described that the antibodies may be polyclonal or monoclonal antibodies. However, it only discloses the preparation process of the polyclonal antibody and its application in the superovulation of domesticated mammals in the specification, and fails to disclose any contents related to the preparation of the monoclonal antibody and its application.

Therefore, since active immunization against inhibin has a negative impact on the conception rate, and the components of AIS used in passive immunization are complex and difficult to make medicines, there exists a need for monoclonal antibodies with clear components that can specifically neutralize inhibin in the field of animal reproduction, especially economic animals.

### Summary of the Invention

The present disclosure relates to an anti-inhibin antibody or antigen-binding portion thereof, which is used in immunoneutralization against inhibin, promoting production and secretion of endogenous FSH, stimulating follicular development and increase ovulation number in female animals.

The present disclosure provides a method for preparing hybridoma cell lines 4D826 and 1E57 expressing anti-inhibin monoclonal antibody, the method comprises:
(1) immunizing animals by immunogens, wherein the immunogens are made by coupling 6-25 fragment of porcine inhibin α subunits (polypeptide sequence as shown in SEQ ID NO.24 in the sequence listing) with carrier protein ;
(2) isolating the splenocytes of the immunized animals and fusing them with appropriate myeloma cells under conditions suitable for producing hybridoma cells;
(3) screening and culturing the hybridoma cells obtained above.

The present disclosure provides an anti-inhibin antibody selected from antibodies 4D8 and 1E5 or an antigen-binding portion thereof.

The present disclosure provides an anti-inhibin antibody or antigen-binding portion thereof, comprising heavy chain CDRs shown in the amino acid sequences of SEQ ID NO. 1, 2, 3, and ight chain CDRs shown in the amino acid sequences of SEQ ID NO. 4, 5, 6; and an anti-inhibin antibody or antigen-binding portion thereof comprising heavy chain CDRs shown in the amino acid sequence of SEQ ID NO. 11, 12, 13, and light chain CDRs shown in the amino acid sequence of SEQ ID NO. 14, 15, 16.

The present disclosure provides an anti-inhibin antibody or antigen-binding portion thereof, comprising a heavy chain variable region of monoclonal antibody 4D8 having the amino acid sequence shown in SEQ ID NO. 7, anda light chain variable region of monoclonal antibody 4D8 having the amino acid sequence shown in SEQ ID NO. 9 ; and/or an anti-inhibin antibody or antigen-binding portion thereof, comprising a heavy chain variable region of monoclonal antibody 1E5 having the amino acid sequence shown inSEQ ID NO. 17, and a light chain variable region of monoclonal antibody 1E5 having the amino acid sequence shown in SEQ ID NO. 19.

The present disclosure provides a nucleic acid molecule encoding the anti-inhibin antibodies or antigen-binding portion thereof as described above, for example, the nucleic acid comprises a nucleic acid sequence shown in SEQ ID NO. 8 that encodes the heavy chain variable region of the monoclonal antibody 4D8 and a nucleic acid sequence shown in SEQ ID NO. 10 that encodes the light chain variable region of the monoclonal antibody 4D8; and/or the nucleic acid comprises a nucleic acid sequence shown in SEQ ID NO. 18 that encodes the heavy chain variable region of the monoclonal antibody 1E5 and a nucleic acid sequence shown in SEQ ID NO. 20 that encodes the light chain variable region of the monoclonal antibody 1E5.

The present disclosure provides an espression vector, the expression vector contains the isolated nucleic acid sequences and expression regulation sequences operably linked to the sequences.

The present disclosure provides an antibody expression system, the antibody expression system is constructed by transfecting the above vector into host cells.

The present disclosure provides a method of preparing recombinant anti-inhibin antibody as described above, comprising the following steps: cultivating an expression system containing the above-mentioned antibody under conditions suitable for expressing the above-mentioned antibody, thereby expressing the above-mentioned antibody; and purifying and isolating the antibody.

The host cells used therein are those prepard by prior art, and may be obtained directly through commercial channels. The culture mediums used in the culturing step are various types of conventional culture mediums. A person skilled in the art can select suitable culture mediums based on experiences to optimize the culture conditions. When the host cells grow to a certain density, an appropriate method can be selected to induce and promote the expression of monoclonal antibodies, and continue to culture the cells. In the above method, the recombinant monoclonal antibody can be expressed in the cell or on the cell membrane, or of course, maybe secreted outside the cell, and then the recombinant protein can be separated and purified by physical, chemical and other characteristics. Obviously, these techniques are well known to those skilled in the art. The above methods include, but are not limited to: denaturation and renaturation of proteins, centrifugation, ultrafiltration, chromatography, HPLC and other single techniques or any combination of several techniques.

The gene sequence of target monoclonal antibodies obtained from the hybridoma cell lines of monoclonal antibodies can show the activity of the monoclonal antibody after constructing an expression vector and recombinantly expressing in CHO cells, and a recombinant anti-inhibin monoclonal antibody is obtained.

The present disclosure provides uses of the above-mentioned anti-inhibin antibody in preparation of medications for promoting animal reproduction, estrus synchronization, conception and birth, embryo transfer, improving ovulation quality, promoting livestock reproduction, or inducing estrus in female livestock; the animals comprises rats, pigs, cattle, and goats.

### Brief Description of Drawings

The accompanying drawings, which are incorporated herein and constitute a part of this specification, illustrate examples consistent with the specification, and serve to explain the principles of the specification together with the specification.
Figure 1 shows the effect of recombinant monoclonal antibody 4D8 on improving the ovulation quality of ICR mice.
Figure 2 shows the effect of recombinant monoclonal antibody 1E5 on improving the ovulation quality of ICR mice.
Figure 3 shows the effect of ascites-derived monoclonal antibody 4D8 on improving the ovulation quality of ICR mice.
Figure 4 shows the effect of ascites-derived monoclonal antibody 1E5 on improving the ovulation quality of ICR mice.
Figure 5 shows the effect of PMSG on improving the ovulation quality of ICR mice.
Figure 6 shows the effect of goat polyclonal antibody on improving ovulation quality of ICR mice.

### Detailed Description of Embodiments

### I. Definition

In the present disclosure, unless otherwise specified, scientific and technical terms used herein have the meanings commonly understood by those skilled in the art. Moreover, the terms related to protein and nucleic acid, chemistry, molecular biology, cell and tissue culture, microbiology, immunology, and operation steps in laboratory used herein are all terms and routine procedures widely used in the corresponding fields. Meanwhile, for a better understanding of the present disclosure, definitions and explanations of related terms are provided below.

The present disclosure provides an antibody specifically binding to inhibin (e.g., monoclonal antibodies) and antigen-binding portion thereof. In specific aspects, tthe present disclosure provides anti-inhibin monoclonal antibodies that specifically bind to inhibin, wherein the anti-inhibin antibodies include variants of parent antibodies. In specific aspects, the present disclosure provides antibodies pecifically binding to inhibin (e.g., mammalian inhibin). In particular aspects, the present disclosure provides anti-inhibin antibodies comprising one or more amino acid residue modifications (e.g., 5-13 amino acid substitutions in the framework region of the variable region of the heavy chain), wherein it retains an affinity to antigen compared to the parent antibody without these modifications. The term "inhibin" refers to any inhibin molecule known to those skilled in the art. For example, the above inhibins can be derived from mammals; for example, inhibins can be derived from mice, pigs, cattle, and goats.

As used herein, unless otherwise stated, the term "about" or "approximately" means within plus or minus 10% of a given value or range. Where integers are required, the term means integers rounding up or down to the nearest whole number within plus or minus 10% of the given value or range.

With respect to polypeptide sequences of chains of antibodies, the phrase "substantially identical" is to be understood that the antibody chain exhibits at least 60%, 65%, 70%, 75%, 80%, 85%, 90%, 95%, 96%, 97%, 98%, 99% or more sequence identity with a reference polypeptide sequence. In the case of nucleic acid sequences, the term is understood that the nucleic acid exhibits at least greater than 60%, 65%, 70%, 75%, 80%, 85%, 90%, 95%, 96%, 97%, 98%, 99% or more sequence identity with the reference nucleic acid sequences.

Sequence "identity" has an art-recognized meaning, and the percentage of sequence identity between two nucleic acid or polypeptide molecules or regions can be calculated using published techniques. Sequence identity can be measured along the entire length of a polynucleic acid or polypeptide, or along a region of the molecule. Although there are many methods of measuring the identity between two polynucleic acids or polypeptides, the term "identity" is well known to person skilled in the art (Carrillo, H. & Lipman, D., SIAM J Applied Math 48:1073 (1988)).

A "substitution" variant is a variant in which at least one amino acid residue is removed from the native sequence and a different amino acid is inserted at the same position. The substitution may be single, where only one amino acid in the molecule is substituted; or the substitution may be multiple, where two or more amino acids in the same molecule are substituted. The multiple substitutions may be at consecutive positions. Likewise, an amino acid may be substituted by multiple residues, where such variants include both substitutions and insertions. An "insertional" variant is a variant in which one or more amino acids are inserted tightly adjacent to an amino acid at a specified position in a native sequence. Tightly adjacent amino acid means those attached to the α-carboxyl or α-amino functional group of the amino acid. A "deletion" variant is a variant in which one or more amino acids have been removed from the native amino acid sequence. Typically, deletion variants have one or two amino acids deleted from a specific region of the molecule.

With respect to the variable domains of antibodies, the term "variable" refers to certain portions of related molecules that differ widely in sequence among antibodies and are used for the specific recognition and binding of a particular antibody to its specific target. However, variability is not evenly distributed throughout the variable domains of antibodies. The variability is concentrated in three segments called complementarity determining regions (CDRs; namely CDR1, CDR2 and CDR3) or hypervariable regions which are located within the variable domains of the light and heavy chains. The more conserved portions of variable domains are called as framework (FR) regions or framework sequences. Each variable domain of native heavy chain and light chain comprises four FR regions, predominantly in a β-folded configuration, the three FR regions are connected by three CDRs, CDRs form a loop, and the loop is connected to the β-folded structures and form partial β-folded structure in some circumstances. The CDRs of each chain are usually connected adjacently by FR regions and contribute to the formation of target binding sites (epitope or determinant) of antibodies with the help of CDRs of other chains (see Kabat et al. Sequences of Proteins of Immunological Interest, National Institute of Health, Bethesda, MD (1987)). As used herein, the numbering of immunoglobulin amino acid residues is performed according to the immunoglobulin amino acid residue numbering system edited by Kabat et al., unless otherwise indicated. A single CDR may have the capability of specifically binding to a cognate epitope.

As used herein, an "antibody fragment" or "antigen-binding portion" of an antibody refers to any portion of a full-length antibody that is less than full-length, but comprises at least a portion of the variable region (e.g., one or more CDRs and/or one or more antibody combining sites), and thus the "antibody fragment" or "antigen-binding fragment" retains binding specificity and at least a part of the specific binding ability of the full-length antibody. Thus, an antigen-binding portion refers to an antibody fragment comprising an antigen-binding portion that binds to the same antigen as the antibody from which the antibody fragment is derived. Antibody fragments comprise antibody derivatives produced by enzymatic treatment of full-length antibodies, as well as antibody derivatives produced synthetically, e.g., recombinantly produced derivatives. Antibodies comprise antibody fragments. Examples of antibody fragments comprise, but are not limited to, Fab, Fab', F(ab')2, single chain Fv (scFv), Fv, dsFv, diabody, Fd and Fd' fragments, and other fragments, including modified fragments (see, for example, Methods in Molecular Biology, Vol 207: Recombinant Antibodies for Cancer Therapy Methods and Protocols (2003); Chapter 1; p 3-25, Kipriyanov). The fragments may comprise multiple chains linked together, for example, by disulfide bonds and/or by peptide linkers. Antibody fragments generally comprise at least or about 50 amino acids, and typically at least or about 200 amino acids. Antigen binding portions include any antibody fragment which, when inserted into an antibody framework (e.g., by substituting corresponding regions), results in an antibody that immunospecifically binds to (i.e. exhibits a Ka of at least or at least about 10⁷-10⁸ M⁻¹) an antigen. A "functional fragment" or "analog of an anti-inhibin antibody" is a fragment or analog that prevents or substantially reduces the binding ability of the receptor to a ligand or initiate signal transduction. As used herein, a functional fragment generally has the same meaning as an "antibody fragment", and, with respect to an antibody, the fragment may refer to a fragment that prevents or substantially reduces the binding ability of the receptor to a ligand or initiate signal transduction, such as Fv, Fab, F(ab')2 and so on. The "Fv" fragment consists of a dimer (VH-VL dimer) formed by a variable domain of a heavy chain and a variable domain of a light chain in a non-covalent association. In this configuration, three CDRs of each variable domain interact with each other to determine a target binding site on the surface of the VH-VL dimer, as is the case with intact antibodies. The six CDRs described above confer target-binding specificity to intact antibodies. However, even a single variable domain (or half of an Fv comprising only 3 target-specific CDRs) may still have the ability to recognize and bind to a target.

As used herein, the term "bispecific antibody" (BsAb) refers to an antibody and/or antigen-binding molecule capable of specifically binding to two different antigenic determinants. Usually, a bispecific antibody and/or antigen-binding molecule comprises two antigen-binding sites, wherein each is specific for a different antigenic determinant. In some embodiments, the bispecific antibody and/or antigen binding molecule is capable of simultaneously binding to two antigenic determinants, and particularly to two antigenic determinants expressed on two different cells.

As used herein, a "monoclonal antibody" refers to a population of identical antibodies, meaning that each individual antibody molecule in the population of monoclonal antibodies is identical to the other antibody molecules. This property is in contrast to that of polyclonal populations of antibodies, the polyclonal population of antibodies comprises antibodies having a variety of different sequences. Monoclonal antibodies can be prepared by many well-known methods (Smith et al. (2004) J. Clin. Pathol. 57, 912-917; and Nelson et al., J Clin Pathol (2000), 53, 111-117). For example, monoclonal antibodies can be prepared by immortalizing B cells, e.g., by fusing with myeloma cells to generate hybridoma cell lines, or by infecting B cells with a virus such as EBV. Recombinant techniques can also be used to prepare antibodies from clonal populations of host cells *in vitro* by transforming host cells with a plasmid carrying an artificial nucleic acid sequences encoding the antibodies.

As used herein, the term "hybridoma" or "hybridoma cell" refers to a cell or cell line resulting from the fusion of antibody-producing lymphocytes and non-antibody-producing cancer cells (typically a myeloma or lymphoma cell). As is known to those skilled in the art, hybridomas can proliferate and continously produce specific monoclonal antibodies. Methods for producing hybridomas are known in the art (see, e.g., Harlow & Lane, 1988). When the term "hybridoma" or "hybridoma cell" is referred to, it also comprises subclones and progeny cells of the hybridoma.

As used herein, a full-length antibody is an antibody having two full-length heavy chains (e.g., VH-CH1-CH2-CH3 or VH-CH1-CH2-CH3-CH4) and two full-length light chains (VL-CL) and a hinge region, such as antibodies produced naturally from antibody-secreting B cells, as well as antibodies produced synthetically having the same domains.

A "humanized" antibody refers to a form of non-human (e.g., mice) antibody that is a chimeric immunoglobulin, immunoglobulin chain, or fragment thereof (e.g., Fv, Fab, Fab', F(ab')2 or other antigen-binding subsequences of an antibody), containing a minimal sequence derived from non-human immunoglobulin. Preferably, the humanized antibody is a human immunoglobulin (recipient antibody) in which residues of the complementarity determining regions (CDRs) of the recipient antibody are replaced by CDR residue substitutions of a non-human species such as mice, rats or rabbits having the desired specificity, affinity and capacity (donor antibody).

In addition, in humanization, it is also possible to mutate amino acid residues in the CDR1, CDR2 and/or CDR3 of the VH and/or VL, thereby improving one or more binding properties (e.g., affinity) of antibodies. Mutations can be introduced, for example by PCR-mediated mutagenesis, and have an impact on the binding or other functional properties of antibodies, can be assessed using the *in vitro* or *in vivo* assays mentioned herein. Typically, conservative mutations are introduced. Such mutations may be amino acid substitutions, additions or deletions. Additionally, there are usually no more than one or two mutations within CDRs. Therefore, the humanized antibody mentioned in the present disclosure also covers the antibodies comprising 1 or 2 amino acid mutations in CDRs.

As used herein, the term "CDR" refers to a complementarity-determining region, and it is known that each heavy and light chain of an antibody molecule has 3 CDRs. CDRs are also called hypervariable regions, present in variable regions of each of heavy chain and light chain of an antibody, and have very high variability sites in the primary structure of the CDRs. In present specification, the CDRs of heavy chain represents CDR1, CDR2 and CDR3 derived from the amino terminal of the amino acid sequence of heavy chain, and the CDRs of light chain represents by CDR1, CDR2, and CDR3 derived from the amino terminal of the amino acid sequence of light chain. These sites are adjacent to each other in the tertiary structure and determine the specificity of the antigen to which the antibody binds.

As used herein, the term "epitope" refers to any antigenic determinant on an antigen to which the paratope of an antibody binds. Epitopic determinants usually comprise chemically active surface types of molecules, such as amino acids or sugar side chains, and usually have specific three-dimensional structural characteristics as well as specific charge characteristics.

As used herein, "specifically binding" or "immunospecifically binding" with respect to an antibody or antigen-binding portion thereof could be used interchangeably in the text and refers to the ability for antibody or antigen-binding portion thereof to form one or more noncovalent bonds to the same type of antigen by noncovalent interactions between antibody and the antibody-binding site of the antigen. The antigen may be an isolated antigen or present in tumor cells. Typically, an antibody immunospecifically binds (or specifically binds) to an antigen with an affinity constant Ka of about or 1×10⁷ M⁻¹ or 1×10⁸ M⁻¹ or more or a dissociation constant (Kd) of 1×10⁻⁷ M or 1×10⁻⁸ M or less). Affinity constants can be measured by standard kinetic methods of antibody reactions, e.g., immunoassay, surface plasmon resonance (SPR) (Rich and Myszka (2000) Curr. Opin. Biotechnol 11:54; Englebienne (1998) Analyst. 123: 1599), isothermal titration calorimetry (ITC), or other kinetic interaction assays known in the art (see, for example, Paul, ed., Fundamental Immunology, 2nd ed., Raven Press, New York, pages 332-336 (1989); see also U.S. Patent No. 7,229,619 which describes exemplary SPR and ITC methods for calculating the binding affinity of antibodies). Instruments and methods for detecting and monitoring association rates in real-time are known and commercially available (see, BiaCore 2000, Biacore AB, Upsala, Sweden and GE Healthcare Life Sciences; Malmqvist (2000) Biochem.Soc.Trans.27 :335).

As used herein, the terms "polynucleic acid" and "nucleic acid molecule" refer to an oligomer or polymer comprising at least two linked nucleic acids or nucleic acid derivatives, including deoxyribonucleic acid (DNA) and ribonucleic acid (RNA) linked together by phosphodiester bonds. As used herein, the term "nucleic acid molecule" is intended to include DNA molecules as well as RNA molecules. A nucleic acid molecule can be single- or double-stranded, and may be cDNA.

As used herein, an isolated nucleic acid molecule is a nucleic acid molecule separated from other nucleic acid molecules that exist in the natural source of the nucleic acid molecule. An "isolated" nucleic acid molecule, such as a cDNA molecule, may be substantially free of other cellular material or culture medium when prepared by recombinant techniques, or substantially free of chemical precursors or other chemical ingredients when chemically synthesized. Exemplary isolated nucleic acid molecules provided herein comprise isolated nucleic acid molecules encoding an antibody or antigen binding portion provided herein.

As used herein, "operably linked" with respect to nucleic acid sequences, regions, elements or domains means that the nucleic acid regions are functionally related to each other. For example, a promoter can be operably linked to a nucleic acid encoding a polypeptide such that the promoter regulates or mediates transcription of the nucleic acid.

As used herein, "expression" refers to a process by which a polypeptide is produced by transcription and translation of a polynucleic acid. Expression levels of a polypeptide can be assessed by any method known in the art, including, for example, methods for determining the amount of polypeptide produced by a host cell. Such methods may comprise, but are not limited to, ELISA for quantification of polypeptides in cell lysates by, Coomassie brilliant blue staining after gel electrophoresis, Lowry protein assay, and Bradford protein assay.

As used herein, a "host cell" is a cell for receiving, maintaining, replicating and amplifying a vector. Host cells can also be used to express polypeptides encoded by vectors. When the host cell divides, the nucleic acid contained in the vector replicates, thereby amplifying the nucleic acids. Host cells may be prokaryotic cells, such as *Escherichia coli*; or lower eukaryotic cells, such as yeast cells- *Pichia pastoris*/*Saccharomyces cerevisiae* or filamentous fungi; or higher eukaryotic cells, such as mammalian cells CHO/293T, various COS cells, HeLa cells, HEK cells such as HEK 293 cells, murine myeloma (NSO) cells, baby hamster kidney (BHK) cells, etc.

As used herein, a "vector" is a replicable nucleic acid from which one or more heterologous proteins can be expressed when the vector is transformed into an appropriate host cell. Vectors comprise those vectors into which a nucleic acid encoding a polypeptide or fragment thereof can be introduced, typically by restriction enzyme digestion and ligation. Vectors also comprise those vectors comprising a nucleic acid encoding a polypeptide. Vectors are used to introduce nucleic acids encoding polypeptides into host cells for amplifying nucleic acids or expressing/displaying polypeptides encoded by nucleic acids. Vectors usually remain episomal, but can be designed to allow integration of a gene or part thereof, into the chromosome of a genome.Vectors are also comtemplated to be artificial chromosomes, such as yeast artificial vectors and mammalian artificial chromosomes. The selection and use of such vectors are well known tothose skilled in the art.

As used herein, vectors also include "viral vector" or " vector of virus". Viral vectors are engineered viruses that are operably linked to foreign genes to transfer (as media or shuttles) foreign genes into cells.

As used herein, an "expression vector" includes a vector capable of expressing DNA, the DNA is operably linked to regulatory sequences such as a promoter region, which is capable of affecting the expression of such DNA fragments. Such additional fragments may include promoter and terminator sequences, and optionally may include one or more origins of replication, one or more selectable markers, enhancers, polyadenylation signals, etc. Expression vectors are generally derived from plasmid or viral DNA, or may contain elements of both. Thus, an expression vector refers to a recombinant DNA or RNA construct, such as a plasmid, phage, recombinant virus or other vector, when introduced into an appropriate host cell, it results in the expression of cloned DNA. Appropriate expression vectors are well known to those skilled in the art, and comprise expression vectors replicable in eukaryotic cells and/or prokaryotic cells, expression vectors that remain episomal, or expression vectors that integrated into the host cell genome. The expression vector used herein refers to bacterial plasmid, yeast plasmid, plant cell virus, mammalian cell virus such as adenovirus, retrovirus, or other vectors well known to those skilled in the art.

As used herein, "treating" an individual suffering from a disease or disease status means that the individual's symptoms are partially or fully alleviated, or remain unchanged after treatments. Thus, treatment includes prevention, treatment and/or cure. Prevention refers to preventing an underlying disease and/or preventing the worsening of symptoms or development of a disease. Treatment also includes any pharmaceutical use of any antibody or antigen-binding portion thereof and compositions provided herein.

### II. Detailed description of embodiments

In one aspect, the present disclosure provides an anti-inhibin antibody or an antigen-binding portion thereof comprising heavy chain CDRs selected from the amino acid sequences of SEQ ID NO. 1, 2, 3, 11, 12, 13 or any variant thereof, and light chain CDRs selected from the amino acid sequences of SEQ ID NO. 4, 5, 6, 14, 15, 16 or any variant thereof.

According to the preceding aspect, the antibody or antigen-binding portion thereof comprises a heavy chain CDR1 selected from the amino acid sequences of SEQ ID NO. 1, 11 or any variant thereof, a heavy chain CDR2 selected from the amino acid sequences of SEQ ID NO. 2, 12 or any variant thereof, a heavy chain CDR3 selected from the amino acid sequences of SEQ ID NO. 3, 13 or any variant thereof, a light chain CDR1 selected from the amino acid sequences of SEQ ID NO. 4, 14 or any variant thereof, a light chain CDR2 selected from the amino acid sequences of SEQ ID NO. 5, 15 or any variant thereof, a light chain CDR3 selected from the amino acid sequences of SEQ ID NO.6, 16 or any variant thereof.

According to the previous aspect, the antibody or antigen-binding portion thereof comprises a combination of CDRs of a heavy chain and a light chain selected from:
(1) heavy chain CDR1, CDR2 and CDR3 comprising the sequences of SEQ ID NO.1, 2, 3 or any variant thereof respectively, and light chain CDR1, CDR2 and CDR3 comprising the sequences of SEQ ID NO. 4, 5, 6 or any variant thereof respectively; or
(2) heavy chain CDR1, CDR2 and CDR3 comprising the sequences of SEQ ID NO. 11, 12, 13 or any variant thereof respectively, and light chain CDR1, CDR2 and CDR3 respectively comprising the sequences of SEQ ID NO. 14, 15, 16 or any variant thereof respectively.

In some embodiments, the above-mentioned antibody or antigen-binding portion thereof comprises a heavy chain variable region having the amino acid sequence selected from SEQ ID NO. 7, 17 or any variant thereof, and a light chain variable region having the amino acid sequence selected from SEQ ID NO. 9, 19 or any variant thereof.

In some embodiments, the above-mentioned antibody or antigen-binding portion thereof comprises a heavy chain variable region of the amino acid sequence of SEQ ID NO. 7 or any variant thereof, and a light chain variable region of the amino acid sequence of SEQ ID NO. 9 or any variant thereof.

In some embodiments, the above-mentioned antibody or antigen-binding portion thereof comprises a heavy chain variable region of the amino acid sequence of SEQ IN NO. 17 or any variant thereof, and a light chain variable region of the amino acid sequence of SEQ ID NO. 19 or any variant thereof.

In one aspect, the present disclosure provides a bispecific or multispecific molecule comprising the antibody or antigen-binding portion thereof of any preceding aspects.

In one aspect, the present disclosure provides a nucleic acid molecule encoding an antibody or antigen-binding portion thereof or bispecific or multispecific molecule according to any of the preceding aspects.

In some embodiments, the nucleic acid molecule comprises a nucleic acid sequence selected from SEQ ID NO. 8, 18 or any variant thereof that encodes an antibody heavy chain variable region, and a nucleic acid sequence selected from SEQ ID NO. 10, 20 or any variant thereof that encodes an antibody light chain variable region.

In some embodiments, the nucleic acid molecule comprises a nucleic acid sequence shown in SEQ ID NO. 8 or any variant thereof that encodes a heavy chain variable region, and a nucleic acid sequence shown in SEQ ID NO. 10 or any variant thereof that encodes a light chain variable region.

In some embodiments, the nucleic acid molecule comprises a nucleic acid sequence shown in SEQ ID NO. 18 or any variant thereof that encodes a heavy chain variable region, and a nucleic acid sequence shown in SEQ ID NO. 20 or any variant thereof that encodes a light chain variable region.

In one aspect, the present disclosure provides an anti-inhibin antibody or antigen-binding portion thereof having sequence identity of at least greater than 60%, 65%, 70%, 75%, 80%, 85% %, 90%, 95%, 96%, 97%, 98%, 99% or higher with the antibody or antigen binding portion thereof according to any of the preceding aspects.

In one aspect, the present disclosure provides nucleic acid molecules encoding the antibody or antigen-binding portion thereof according to any of the preceding aspects, or nucleic acid molecules having at least greater than 60%, 65%, 70%, 75%, 80%, 85%, 90% , 95%, 96%, 97%, 98%, 99% or higher sequence identity.

In one aspect, the present disclosure provides a vector comprising a nucleic acid according to any of the preceding aspects.

In one aspect, the present disclosure provides a cell comprising a vector according to any of the preceding aspects.

In one aspect, the present disclosure provides an expression system constructed by introducing an expression vector comprising the aforementioned nucleic acid into a host cell.

In some embodiments, the expression vector comprises any one selected from bacterial plasmids, yeast plasmids, plant cell viruses, and mammalian cell viruses (such as adenovirus or retrovirus).

In some embodiments, the expression vector is pcDNA3.1(+).

In some embodiments, the host cell is a prokaryotic host cell or a eukaryotic host cell.

In some embodiments, the prokaryotic host cell is a bacterial cell.

In some embodiments, the aforementioned bacterial cells are *Escherichia coli.*

In some embodiments, the eukaryotic host cell is selected from any one of fungi, plants, insects, and mammals.

In some embodiments, the fungal eukaryotic host cell is selected from any one of yeast (such as *Pichia pastoris* and *Saccharomyces cerevisiae*) and filamentous fungi.

In some embodiments, the mammalian eukaryotic host cell is selected from any one of Chinese hamster ovary (CHO) cells, murine myeloma (NSO) cells, baby hamster kidney (BHK) cells, and human embryonic kidney (HEK) cells (such as HEK293 cells).

In some embodiments, the host cell is the Chinese hamster ovary (CHO) cell.

According to the expression system of the previous aspect, the method of introducing the expression vector into the host cell is selected from transfection, transformation or infection .

In some embodiments, the expression vector is introduced into the host cell by transfection.

In some embodiments, the transfection includes: electroporation transfection, calcium phosphate transfection, liposome transfection, protoplast fusion transfection, microinjection, electroporation, gene gun, cationic polymerization and viral vector infection.

In one aspect, the present disclosure provides a composition comprising the antibody or antigen-binding portion thereof, the bispecific or multispecific molecule, nucleic acid, vector and cell of any of the previous aspects

In one aspect, the present disclosure provides uses of antibody or antigen-binding portion thereof, bispecific or multispecific molecules, nucleic acids, vectors, cells, and compositions of any of the preceding aspects in preparation of medications for for promoting animal reproduction, estrus synchronization, conception and birth, embryo transfer, improving ovulation quality, promoting livestock reproduction, or inducing estrus in female livestock..

In some embodiments, the aforementioned animals include mice, pigs, cattle, and goats.

In one aspect, the present disclosure provides methods for promotomg animal reproduction, estrus synchronization, conception and birth, embryo transferring, improving ovulation quality, promoting livestock reproduction, or inducing estrus in female livestock, the method comprises a step of administering the aforementioned antibodies or antigen-binding portions thereof to animals.

In some embodiments, the dosage of administering the medication is 0.1~1,000 µg/kg.

In some embodiments, the medications are administered by intraperitoneal, intramuscular, or subcutaneous injections.

In some embodiments, the antibody or antigen-binding portion thereof and the bispecific or multispecific molecule are administered at a dose of 0.1-1,000 µg/kg by intraperitoneal, intramuscular or subcutaneous injections.

The antibodies herein, and derivatives, fragments, analogs and homologues thereof, can be incorporated into pharmaceutical compositions suitable for administration. The principles and considerations involved in the preparation of such compositions, as well as guideline for selecting components, are well known in the art.

Such compositions generally comprise an antibody and a pharmaceutically acceptable carrier. When using antibody fragments, minimal inhibitory fragments that specifically bind to the binding domain of the target protein may be preferred. For example, based on the variable region sequences of antibodies, peptide molecules can be designed to retain the bingding ability to target protein sequences. Such peptides can be chemically synthesized and/or produced by recombinant DNA techniques (see, e.g., Marasco et al., Proc. Natl. Acad. Sci. USA, 90:7889-7893 (1993)).

As used herein, the term "pharmaceutically acceptable carrier" is intended to include any and all of solvents, dispersion media, coatings, antibacterial agents and antifungal agents, isotonic agents and absorptiondelaying agents, etc., which are compatible with administering medication. Suitable pharmaceutically acceptable carriers are described in the latest edition of Remington's Pharmaceutical Sciences, a standard reference bookin the field, which is incorporated herein by reference. Preferred examples of such carriers or diluents include, but are not limited to, water, saline, Ringer's solution, glucose solution, and 5% human serum albumin. Liposomes and non-aqueous vehicles, such as immobilized oil, may also be used. The use of such media and agents for pharmaceutically active substances is well known in the art. Their use in compositions is envisioned except for any conventional media or reagents with which the antibodies are incompatible.

The pharmaceutical composition in the above embodiments is formulated to be compatible with its intended routes of administration. Examples of routes of administration comprise parenteral, e.g., intravenous, intradermal, subcutaneous, oral (e.g., inhalation), transdermal (i.e., topical), transmucosal, and rectal administrations. Solutions or suspensions for parenteral, intradermal or subcutaneous administrations may comprise the following ingredients: sterile diluents for injection such as water, saline solution, immobilized oil, polyethylene glycols, glycerin, propylene glycol or other synthetic solvents; antibacterial agents such as benzyl alcohol or methyl p-hydroxybenzoate; antioxidants such as ascorbic acid or sodium bisulfite; chelating agents such as ethylenediaminetetraacetic acid (EDTA); buffers such as acetates, citrates or phosphate, and agents to adjust osmotic pressure, such as sodium chloride or dextrose. The pH can be adjusted with acids or bases, such as hydrochloric acid or sodium hydroxide. A parenteral preparation can be packaged in ampoules, disposable syringes or multiple dose vials made of glass or plastic.

Pharmaceutical compositions suitable for injection uses comprise sterile aqueous solutions (water soluble herein) or dispersions and sterile powders for the instant preparation of sterile injectable solutions or dispersion. For intravenous administration, suitable pharmaceutically acceptable carriers comprise normalsaline, bacteriostatic water, Cremophor EL^{™} (BASF, Parsippany, N.J.) or phosphate buffered saline (PBS). In all cases, the composition must be sterile and should be flowbable to the extent that is easy to inject. It must be stable under the conditions of manufacture and storage and must be protected from the contamination of microorganisms such as bacteria and fungi. The carrier may be a solvent or dispersion medium comprising, for example, water, ethanol, polyol (for example, glycerol, propylene glycol, and liquid polyethylene glycol, etc.), and a suitable mixture thereof. The desired particle size can be maintained in the case of dispersions, for example, by the use of coatings such as lecithin, and proper fluidity can be remained by the use of surfactants. Prevention of microbial effects can be brought about by various antibacterial and antifungal agents, for example, methyl p-hydroxybenzoate, chlorobutanol, phenol, ascorbic acid, thiomersal, etc. In many cases it will be preferable to include isotonic agents, for example, sugars, polyalcohols (such as mannitol, sorbitol), sodium chloride in the composition. Prolonged absorption of the injectable compositions can be brought about by comprising in the composition an agent delaying absorption, for example, aluminum monostearate and gelatin.

As required, sterile injectable solutions can be prepared by incorporating an antibody in a required amount into an appropriate solvent with one or a combination of ingredients enumerated above, followed by filtering and sterilization. Generally, dispersions are prepared by incorporating the antibody into a sterile vehicle that cpmprises a basic dispersion medium and other required ingredients from those enumerated above. In the case of sterile powders for the preparation of sterile injectable solutions, the methods of preparation are vacuum-drying and freeze-drying to obtain a powder comprising active ingredients and any additional desired ingredient from a sterile-filtered solution of these ingredients as previously described.

For administration by inhalation route, compounds are delivered as an aerosol spray from a pressurized container or dispenser or sprayer containing a suitable propellant, such as carbon dioxide.

Systemic administration can also be performed by transmucosal or transdermal routes. For transmucosal or transdermal administration, penetrants appropriate to the barrier to permeate are used in the preparation. Such penetrants are generally known in the art and comprise, for example, detergents, bile salts and fusidic acid derivatives for mucosal administration. Transmucosal administration can be accomplished through the use of nasal sprays or suppositories. For transdermal administration, one or more antibodies may be formulated into pastes, ointments, gels, or creams as generally known in the art.

The compounds may also be prepared in the form of suppositories (e.g., with conventional suppository bases such as cocoa butter or other glycerides) or retention enemas for rectal delivery.

In one embodiment, antibodies are prepared with carriers thamay protect them from rapid elimination from the body, such as slow/controlled release preparations, comprising implants and microencapsulated delivery systems. Biodegradable, biocompatible polymers may be used, such as ethylene vinyl acetate, polyanhydrides, polyglycolic acid, collagen, polyorthoesters, and polylactic acid. Methods for preparation of such formulations will be apparent to those skilled in the art.

It is especially advantageous to prepare parenteral compositions in dosage unit form for ease of administration and uniformity of dosage. The dosage unit form as used herein refers to physically separable units suited as unitary dosages for the subjects awating treatment; each unit comprises a predetermined quantity of one or more antibodies calculated to produce the desired therapeutic effect in association with the required pharmaceutical carrier. The specifications for dosage unit forms of the above embodiments are dictated by and directly dependent on: the unique characteristics of the antibody, the particular therapeutic effects to be achieved, and the limitations inherent in the field of formulation of such antibodies for treatment of individuals.

The pharmaceutical composition can be presented in a container, package, or dispenser together with instructions for administration.

The preparation described herein may also comprise more than one antibody, preferably those antibodies with complementary activities that do not adversely affect each other, it depends on the particular conditions to be treated. Alternatively or in addition, a composition may, for example, comprise an agent that enhances its function, such as cytotoxic agents, cytokines, chemotherapeutic agents, or growth inhibitors. Such molecules are suitably present in combination in an effective amount for the intended purpose. For example, they may be present in combination in a kit or in use.

In one embodiment, one or more antibodies may be administered in combination therapies, i.e., combined with other agents, such as therapeutic agents, (which are used for the treatment of pathological conditions or disorders, such as various forms of cancer, autoimmune disorders, and inflammatory diseases). The term "combination" herein means that the agents are substantially administered synchronously, simultaneously or sequentially. If administered sequentially, when administration of the second compound is initiated, the first of the two compounds is still preferably detectable at the site of treatment at an effective concentration. In one instance, "combination" can also be a kit comprising both an antibody of the present disclosure and another therapeutic agent.

For example, combination therapies may comprise one or more antibodies described herein and one or more additional therapeutic agents (e.g., one or more cytokines and growth factor inhibitors, immunosuppressants, anti-inflammatory agents, metabolic inhibitors , enzyme inhibitors, and/or cytotoxins or cytostatic agents, as described in more detail below) are co-formulated and/or co-administered. Such combination therapies may advantageously administer lower doses of the therapeutic agents, thereby avoiding possible toxicity or complications associated with various monotherapies.

For purposes of clearly and concisely describing the present invention, features are described herein as a part of the same embodiment or separated embodiments. However, it will be understood that the scope of the present disclosure may include some embodiments with all features or a combination of some features described herein.

### Examples

### Example 1: Preparation of Hybridoma Cells of Anti-inhibin Monoclonal Antibody

### 1. Preparation of Immunogen

After searching the Uniprot database, it was found that the homology of the 6-25 fragment of inhibin α subunits in mice, rats, pigs, cattle and goats was 100%, therefore this fragment was selected as an antigenic peptide to screen for simultaneously acting on mice, rats, pigs, cattle, and goats for producing anti-inhibin monoclonal antibodies. The above antigenic peptide fragments were coupled to bovine serum albumin (BSA), and the conjugate was used as an immunogen to enhance the immunogenicity of the antigenic peptide.

### 2. Immunization of animals

Three 6-8-week-old female BALB/c mice were selected, all mice were of SPF grade, and were perchased from Zhejiang Weitong Lihua Experimental Animal Technology Co., Ltd.. The immunological reagent was Freund's adjuvant, which was purchased from Shanghai Beyotime Biotechnology Co., Ltd.. The immunization scheme was shown in Table 1:

**Table 1. Immunization route and cycle**

| **Immunization procedure** | **Route** | **Dosage(µg)** | **Adjuvant(µl)** | **Immunization interval (w)** |
|---|---|---|---|---|
| First immunization | Subcutaneous injection | 100 | 100 | 0 |
| Booster immunizaition | Subcutaneous injection | 50 | 50 | 3 |
| Rush immunization | Intraperitoneal injection | 100 | - | 3 |

### 3. Preparation of hybridoma cells

### 3.1 Preparation before fusion

### (1) Extraction of Macrophage:

The immunized mice were killed by pulling their necks, soaked in alcohol for 5-10 minutes,taken out and turned upside down, placed on the dissecting table, where the limbes were fixed, the abdomen was scrubbed with 75% alcohol again, and the abdominal skin was cut to expose the peritoneum under aseptic conditions, the peritoneum was wiped and disinfected with 75% alcohol;

5ml of sterile DMEM medium was drawn usng a 10ml syringe, after the peritoneum was lifted with tweezers, the medium was injected into the abdominal cavity of the mouse, simultaneously the abdomen was kneaded or the thighs were lifted with fingers from both sides so as to allow the culture medium to flow fully within the abdominal cavity. When the liquid turns yellow after 5 mins, the peritoneum was lifted again with tweezers and the liquid was drawn with a syringe to obtain a medium containing macrophages, after centrifuging at 1,000 rpm for 5min, washing with PBS, the macrophages were suspended with 10ml HAT medium, and counted for later use.

### (2) Culturing and collection of myeloma cells sp2/0

The cells cultured in the T75 culture flask (grown well and in the logarithmic phase) were lightly patted and washed once with PBS, then suspended with 10ml of PBS, and counted for later use.

### (3) Preparation of splenocyte

For the mice that the macrophages were extracted, the peritoneum was cut open by aseptic operation, the spleen was taken out, placed in incomplete medium, and crushed by pressing to obtain a large number of cells. After centrifuging at 1,000 rpm for 5min, and washing with PBS for 2-3 times, the cells were finally suspended with 10ml PBS. an appropriate amount were taken for counting, and set aside for later use.

### 3.2 Cell fusion

The previously prepared myeloma cells and spleen cells were taken, mixed ttogether at a ratio of 1:10, washed once with an incomplete medium in a 50ml centrifuge tube. After centrifuging at 1,000 rpm for 8min, the supernatant was discarded and the residual liquid was sucked up, the bottom of the centrifuge tube was flicked to loosen the cell pellet;

1ml of 50% PEG (pH8.0) preheated to 40°C was added into a pipette within 60s, and stirred gently during the addition process;

20ml of preheated incomplete medium was added into a 10ml pipette within 90s, and set aside at room temperature for 10min;

After centrifuging at 1,000 rpm for 6min, the supernatant was discarded;

After adding 5ml of HAT medium, the cell precipitate were gently blown and aspirated to suspend and mix uniformly , then macrophages are added and add HAT medium to 60ml;

After Aliquoting in 96-well cell culture plate, 200µl per well, the celles were cultivated in an incubator at 37°C, in an atmosphere of 5% CO₂;

After 7 days, the HAT medium was replaced with HT medium.

### 3.3 Monoclonalization of hybridoma cells

The 6-25 fragment of inhibin α-subunits coupled to chicken ovalbumin (OVA) were diluted to a sodium bicarbonate solution having a pH9.6 at a concentration of 10 µg/ml, and 100 µl per well was added to the microtiter plate (96 well plate). The plate was set aside overnight at 2-8°C overnight, and taken out for washing and then patted dry next day, 5% BSA was added into the plate to block, 200µl per well, and incubated at 37°C for 1h;

The plate was washed and patted dry. 100 µl of the fused culture supernatant was taken under aseptical conditions, added to the microtiter plate, and incubated at 37°C for 1h;

After washing the plate, secondary antibody (goat anti-mouse, HRP-labeled) was added, 100 µl per well, and incubated at 37°C for 1h;

After washing the plate, chromogenic solution was added ino the plate, 100 µl per well, and incubated at 37°C for 5-10 minutes. 50 µl of stop solution was added into the plate, and the absorbance value was read at a wavelength of 460 nm.

The wells having an absorbance value of greater than 0.5 were selected, and the cells were transferred to a 24-well plate (a total of 38 positive wells were obtained, wherein the positive rate was low, and the data of the first round was too large to display herein). The plate was continouly cultured in the incubator at 37°C in an atmosphere of 5% CO₂;

After 5-7 days, the supernatant of cells cultured in the 24-well plate were detected, re-screened in the same way to confirm the positive well situation (the value is not shown here). The cells in the 15 positive wells having a highest absorbance value were selected, transferred to a 6-well plate and continuouly cultured in the incubator at 37°C in an atmosphere of 5% CO₂. The remaining positive clones were directly preserved as seeds and stored in liquid nitrogen.

The cells in the 6-well plate were subjected to two rounds of subcloning screening, and a total of 6 subclones were obtained, wherein the cell culture supernatants of 4D826 and 1E57 had the highest titers, and the titer test results were shown in Table 2.

**Table 2 Titer test of cell culture supernatants of hybridoma cell lines 4D826 and 1E57**

| Dilution ratio/sample | 4D826 culture supernatant | | 1E57 culture supernatant | |
|---|---|---|---|---|
| 200 | 3.019 | 2.551 | 3.308 | 3.248 |
| 400 | 2.483 | 2.595 | 2.804 | 2.358 |
| 800 | 2.068 | 1.951 | 2.918 | 2.078 |
| 1600 | 1.343 | 1.258 | 2.284 | 1.904 |
| 3200 | 0.822 | 0.932 | 1.686 | 1.426 |
| 6400 | 0.593 | 0.544 | 1.285 | 0.831 |
| 12800 | 0.403 | 0.354 | 0.823 | 0.648 |
| 25600 | 0.248 | 0.265 | 0.613 | 0.398 |
| 51200 | 0.174 | 0.182 | 0.359 | 0.256 |
| 102400 | 0.139 | 0.136 | 0.263 | 0.189 |
| Control well BSA | 0.08 | 0.08 | 0.079 | 0.081 |

### 4. Subtype analysis of anti-inhibin monoclonal antibodies

Using the monoclonal antibody subtype identification kit from Friendbio Science & Technology (Wuhan) Co., Ltd., the antibody subtypes secreted by two hybridoma cell lines 4D826 and 1E57 were identified according to the instructions. The results showed that both cell lines secreted immunoglobulin, both of heavy chain subtypes were IgG1 and both of light chain subtypes were Kappa.

### 5. Preparation of Ascites

0.5 ml of paraffin oil (purchased from Sigma Co.) was injected into the abdominal cavity of mice a week in advance. One week after the sensitization, the hybridoma cells 4D826 and 1E57 growing vigorously were centrifuged, after discarding the medium, the cell precipitate was resuspended with PBS or incomplete medium, and a cell concentration was adjusted to to 2×10⁷ cells/ml. 0.1ml of cell suspension was injected into the abdominal cavity of mice. After 7-10 days, the abdominal cavity of the mouse were obviously enlarged. At this time, the ascites can be collected directly by killing the mice. Cell debris and oil in ascites were removed by centrifuging at 8,000 rpm for 20 min at 4°C, then an equal volume of glycerol was added and stored at -20°C. The titer of ascites-derived monoclonal antibodies can reach 1: 4,000,000 by indirect ELISA assay.

### Example 2: Cloning to obtain the variable region coding sequence of anti-inhibin monoclonal antibody and vector construction

### 1. Gene sequencing and sequence synthesis

### (1) Determination of the heavy chain variable region and light chain variable region of monoclonal cell lines 4D826 and 1E57

The two monoclonal cell lines 4D826 and 1E57 were sent for sequencing when the titer of the above cell culture supernatants reaches 1:25,600.

The amino acid sequence of the heavy chain variable region of the monoclonal antibody 4D8 is shown in SEQ ID NO. 7, its nucleic acid sequence is shown in SEQ ID NO. 8, and its CDR1, CDR2 and CDR3 are shown in SEQ ID NO. 1, 2, 3 respectively.

<-------------------FR1------------------>                    CDR1                <----------FR2---------->
CDR2                <----------
QVQLKQSGPSLVQPSQSLSITCTVSGFSLTTCGVNWVRQSPGKGLEWLGVIWRGGS
TDYNAAFMSRMSITK
-------FR3-------------------------------------->          CDR3          <------FR4------->
DNSKSQVFFKMNSLPADDTAKYYFAKRKILLNGTVPTGQGTTLTVSS

### Nucleic acid sequence

CAGGTGCAGCTGAAGCAGTCCGGACCCTCCCTGGTGCAGCCCTCCCAGTCCCTGT
CCATCACCTGCACCGTGTCCGGATTCTCCCTGACCACCTGCGGAGTGAACTGGGT
GAGACAGTCCCCCGGAAAGGGACTGGAGTGGCTGGGAGTGATCTGGAGAGGAGG
ATCCACCGACTACAACGCCGCCTTCATGTCCAGAATGTCCATCACCAAGGACAACT
CCAAGTCCCAGGTGTTCTTCAAGATGAACTCCCTGCCCGCCGACGACACCGCCAA
GTACTACTTCGCCAAGAGAAAGATCCTGCTGAACGGAACCGTGCCCACCGGACAG
GGAACCACCCTGACCGTGTCCTCC

The amino acid sequence of the light chain variable region of the monoclonal antibody 4D8 is shown in SEQ ID NO. 9, its nucleic acid sequence is shown in SEQ ID NO.10, and its CDR1, CDR2 and CDR3 are shown in SEQ ID NO. 4, 5, 6 respectively.

<-------------------FR1----------------->          CDR1        <-----------FR2----------->     CDR2     <--
------------------------FR3--
DIQMTQTTSSLSASLGDRVTISCRASQDISNYLNWYQQKPDGTVKLLIYYTSRFHSG
VPSRFSGSGSGTDYSLT
------------------------>          CDR3       <-----FR4----->
ISNLEQEDIATYFCQQGYSLPPTFGGGTKLEIK

### Nucleic acid sequence

GACATCCAGATGACCCAGACCACCTCCTCCCTGTCCGCCTCCCTGGGAGACAGAG
TGACCATCTCCTGCAGAGCCTCCCAGGACATCTCCAACTACCTGAACTGGTACCAG
CAGAAGCCCGACGGAACCGTGAAGCTGCTGATCTACTACACCTCCAGATTCCACT
CCGGAGTGCCCTCCAGATTCTCCGGATCCGGATCCGGAACCGACTACTCCCTGACC

ATCTCCAACCTGGAGCAGGAGGACATCGCCACCTACTTCTGCCAGCAGGGATACT
CCCTGCCCCCCACCTTCGGAGGAGGAACCAAGCTGGAGATCAAG

The amino acid sequence of the heavy chain variable region of the monoclonal antibody 1E5 is shown in SEQ ID NO. 17, its nucleic acid sequence is shown in SEQ ID NO. 18, and its CDR1, CDR2, CDR3 are shown in SEQ ID NO. 11, 12, 13 respectively.

<-------------------FR1------------------>                CDR1              <------------FR2----------->
CDR2                <-----
EVKLVESGPELKKPGETVKISCKASGYTFTDYSMHWVKQAPGKGLKWMGWINTET
GEPTYADDFKGRFAF
--------FR3------------------------------------>          CDR3          <-------FR4------->
SLETSASTAYLQINNLKNEDTATYFCAYYGPPTYSLDYWGQGASVTVSS

### Nucleic acid sequence

GAGGTGAAGCTGGTGGAGTCCGGACCCGAGCTGAAGAAGCCCGGAGAGACCGTG
AAGATCTCCTGCAAGGCCTCCGGATACACCTTCACCGACTACTCCATGCACTGGGT
GAAGCAGGCCCCCGGAAAGGGACTGAAGTGGATGGGATGGATCAACACCGAGAC
CGGAGAGCCCACCTACGCCGACGACTTCAAGGGAAGATTCGCCTTCTCCCTGGAG
ACCTCCGCCTCCACCGCCTACCTGCAGATCAACAACCTGAAGAACGAGGACACCG
CCACCTACTTCTGCGCCTACTACGGACCCCCCACCTACTCCCTGGACTACTGGGGA
CAGGGAGCCTCCGTGACCGTGTCCTCC

The amino acid sequence of the light chain variable region of the monoclonal antibody 1E5 is shown in SEQ ID NO. 19, its nucleic acid sequence is shown in SEQ ID NO.20, and its CDR1, CDR2, CDR3 are shown in SEQ ID NO.14, 15, 16 respectively.

<-------------------FR1----------------->          CDR1          <------------FR2----------->     CDR2     <--

------------------------FR3--

DIQMTQTTSSLSASLGDRVTISCRASQGISNYLNWYQQKPDGTVKLLIYYTSRLLSG

VPSRFSGSGSGTDYSLT

------------------------>          CDR3          <-----FR4----->

ISNLEQEDIATYFCQQGYTLPFTFGSGTKLEIK

### Nucleic acid sequence

GACATCCAGATGACCCAGACCACCTCCTCCCTGTCCGCCTCCCTGGGAGACAGAG
TGACCATCTCCTGCAGAGCCTCCCAGGGAATCTCCAACTACCTGAACTGGTACCAG
CAGAAGCCCGACGGAACCGTGAAGCTGCTGATCTACTACACCTCCAGACTGCTGT
CCGGAGTGCCCTCCAGATTCTCCGGATCCGGATCCGGAACCGACTACTCCCTGACC
ATCTCCAACCTGGAGCAGGAGGACATCGCCACCTACTTCTGCCAGCAGGGATACA
CCCTGCCCTTCACCTTCGGATCCGGAACCAAGCTGGAGATCAAG

### (2) Determination of signal peptides and constant sequences of recombinant antibodies

The amino acid sequences of the signal peptides of the heavy chain and the light chain are SEQ ID NO.21; the amino acid sequences of the constant regions of the heavy chain and the light chain are SEQ ID NO.22 and SEQ ID NO.23, respectively.

The above sequences are combined according to certain requirements to form the full-length sequences of the heavy chain and antibody light chain of recombinant monoclonal antibodies 4D8 and 1E5.

### 2. Construction of a recombinant expression vector

The vector pcDNA3.1(+) was carried out a double digestion reaction with the target genes of 4D8 and 1E5 respectively using restriction enzymes Nhe I and Xho I. The system was set forth in Table 3 as below:

**Table 3 enzyme digestion system**

| Component | Volume |
|---|---|
| pcDNA3.1/target gene | 10µl |
| Nhe I | 1.5µl |
| Xho I | 1.5µl |
| 10X Buffer | 5µl |
| ddH₂O | Make up to 50µl |

The prepared reaction solutions were put into a 1.5ml centrifuge tube, sealed and bathed in water at 37°C for 6h, and the digested products were taken for agarose gel electrophoresis experiment.

Using the Agarose Gel DNA Recovery Kit (purchased from Tiangen Biochemical Technology (Beijing) Co., Ltd., Article No. DP209), the target DNA band in the agarose gel was recovered after electrophoresis according to the instructions.

The target fragments of the heavy chain and light chain were linked to the cleaved pcDNA3.1 (+) vector using T4 DNA ligase respectively, the reaction system was shown in the instructions.

Transformation of the linked products was carried out by reference to the instructions of Tiangen DH5a competent cell. Positive clones were screened out for expanding cultivation. The plasmid extraction kit was used to extract the plasmid according to the instruction.

### 3. Transient transfection of CHO cells to express recombinant anti-inhibin monoclonal antibodies

(1) Cultivation of CHO-s cells: under conventional culture conditions in a 125ml shake flask, when the cell density increased to 1×10⁷ cells/ml, the cells were seeded into a new 125ml shake flask at an initial density of 2×10⁵ cells/ml, and cultured at 37°C in an atmosphere of 5% CO₂. After 2 days, when the density increased to 5×10⁵ cells/ml, transient transfection was carried out according to the instructions of transfection reagent lipo 2000;
(2) After 7 days, the cell supernatant was collected, cells and debris were removed by centrifuging at 5,000 rpm for 30min. After being filted at 0.45µm and temporarily stored at 4°C, the cell supernatant was used for subsequent purification of antibodies.

### Example 3: Preparation of Goat Polyclonal Antibodies

### 1. Preparation of Inhibin Antigen

The 6-25 fragment of porcine inhibin α subunits (having the amino acid sequence shown in SEQ ID NO.24 in the sequence listing) was synthesized by conventional techniques, and coupled to BSA through the N-terminal.

### 2. Immunization:

For the first immunization, after emulsifying 2 mg of conjugated antigen with Freund's complete adjuvant at a ratio of 1:1, a castrated male goat was subcutaneously immunized in the neck. Freund's incomplete adjuvant was replaced for the second immunization. One week after the fifth immunization, a small sample of serum was taken for determining the titer by ELISA assay; when the titer met the requirements, whole blood was taken after a single booster immunization. The results were shown in Table 4, and the anti-serum titer reached greater than 1:64,000.

**Table 4 Titer determining of goat polyclonal antibodies**

| Dilution fold/sample | Serum of goat polyclonal antibodies | |
|---|---|---|
| 250 | 2.625 | 2.646 |
| 1,000 | 2.331 | 2.411 |
| 4,000 | 1.670 | 1.691 |
| 16,000 | 0.795 | 0.785 |
| 64,000 | 0.240 | 0.235 |
| 256,000 | 0.164 | 0.081 |
| 1,024,000 | 0.050 | 0.059 |
| Control well BSA | 0.054 | 0.078 |

### Example 4: Purification of Antibodies

The antibodies were purified by Protein G affinity chromatography. The samples to be purified comprised the ascites prepared in Example 1, the culture supernatant of transient cells prepared in Example 2, and the goat polyclonal antibodies prepared in Example 3. Specific steps were set forth in as follows:
A. washing the Protein G affinity chromatography column using 3-5 times of column volumes of purified water;
B. washing the Protein G affinity chromatography column using 3-5 times of column volume of 20mM sodium phosphate buffer (pH7.0);
C. pumping the desired purified samples into the chromatographic column;
D. performing an elution process with 100mM citric acid buffer (pH2.7), collecting the elution peak; and neutralizing the collection with 1M Tris buffer (pH9.0);
E. desalting by dialysis with 10mM PB8.0 buffer;
F. Preliminarily identifing antibodies by SDS-PAGE electrophoresis, the results showed that, two target bands at 50kD and 25kD were present in all of the monoclonal antibody prepared from purified ascites, the recombinant monoclonal antibody from transiently transfecting cells, and the goat polyclonal antibody, respectively. The purity were respectively greater than 90%. Similarly, as detected by indirect ELISA, when the concentration of the purified monoclonal antibodies was 1g/L, the titer was 1:1,000,000, while the titer of 1g/L goat polyclonal antibody was 1:16,000.

### Example 5: Determination of Antibody Dosage using Superovulation Experiment in Mice

After a literature review (Ulcova-Gallova Z, Babcova K, Micanova Z, Bibkova K, Rumpik D. Hyperstimulation syndrome: the levels of inhibin A and B in sera and follicular fluids. Gynecol Endocrinol. 2014; 30 (4): 298- 301), it was known that the inhibin content in the human body was about 2.5 µg/L, and the content may increase to greater than 5 µg/L when the follicles were well developed. On this basis, it was estimated that the inhibin content in the body was 5-10 µg according to the weight of a mouse of 25 g. In order to investigate whether there was a correlation between the dosage and the number of ovulations, four of concentration gradients were set up in the antibody group, which were 0.002 µg, 0.02 µg, 0.2 µg, and 20 µg per mouse respectively, in other words, 0.1 µg/kg, 1 µg/kg, 10 µg/kg, and 1,000 µg/kg. Referring to the established dosage scheme of PMSG, the dosage was set at 10 IU/mouse.

5-week-old female ICR mice were selected, and randomly divided into 22 groups, 5 ICR mice in each group, specifically comprising: four groups of recombinant monoclonal antibody 4D8, four groups of recombinant monoclonal antibody 1E5, four groups of ascites-derived monoclonal antibody 4D8, and four groups of ascites-derived monoclonal antibody 1E5; four groups of goat polyclonal antibody, and standard product PMSG; normal saline was used as a negative control. The above-mentioned dosage of antibodies and 10 IU PMSG were respectively injected intraperitoneally at 5:00 p.m. on Monday, and 10 IU HCG was also injected intraperitoneally 48h later. Oocytes were dissected and counted at 9:00 a.m. on Thursday. The results were shown in Table 5. It can be seen that the low-dosage group (0.1 µg/kg) had poorer ovulation-stimulating effects than that of the PMSG group, and the middle-dosage two groups (1 µg/kg, 10 µg/kg) can significantly increase ovulation, but continuosly increase on dosage (high-dosage group, 1,000 µg/kg) did not promote ovulation at the same time; it can also be seen that the monoclonal antibody group had little effects on the number of ovulation under the same dosage, 0.02µg/mouse (the dosage of 1µg/kg) can significantly increase the number of ovulation compared with PMSG, so the following experiments related to mice were temporarily carried out with the dosage of 0.02 µg/mouse compared with the dosage of 10IU PMSG, but it cannot be ruled out that, the dosage would be possibly adjusted with the deepening of research.

**Table 5 Effects of different monoclonal antibodies and dosages on the number of ovulation in mice (ovulation /mouse)**

| Dosage | 0.1 µg/kg | 1 µg/kg | 10 µg/kg | 1,000 µg/kg |
|---|---|---|---|---|
| Recombinant 4D8 | 10.1±2.59 | 22.1±1.54 | 21.6±1.92 | 21.3±2.01 |
| Recombinant 1E5 | 11.3±2.28 | 24.2±2.03 | 22.4±1.23 | 22.7±1.49 |
| Ascites-derived 4D8 | 9.4±2.61 | 22.2±1.61 | 20.8±2.00 | 20.9±2.13 |
| Ascites-derived 1E5 | 10.6±2.62 | 23.9±1.95 | 22.3±1.86 | 23.4±1.75 |
| Goat polyclonal antibodies | 5.1±2.87 | 12.3±2.32 | 10.5±1.99 | 11.1±1.89 |
| PMSG | At 10IU, the number of ovulation was 18.9±2.48 | | | |
| Normal saline | 0 | | | |

### Example 6: Effects of different administration routes on ovulation in mice

In the superovulation experiments of mice, the medications was generally administered intraperitoneally, but considering the limitations of its application in large animals, this experiment mainly depended on whether the two routes of administration, subcutaneous and intramuscular injections, can bring about the same effects.

5-week-old female ICR mice were selected and randomly divided into 17 groups, 5 ICR mice in each group, specifically comprising: three groups of 1 µg/kg recombinant monoclonal antibody 4D8, three groups of 1 µg/kg recombinant monoclonal antibody 1E5, three groups of 1 µg/kg ascites-derived monoclonal antibody 4D8, three groups of ascites-derived monoclonal antibody 1E5, three groups of 1 µg/kg goat polyclonal antibody, and 10IU PMSG; normal saline was used as a negative control.0.02 µg antibody and 10 IU PMSG were respectively injected intraperitoneally, subcutaneously, and intramuscularly at 5:00 p.m. on Monday. 48h later, 10 IU HCG was also injected intraperitoneally. Oocytes were dissected and counted at 9:00 a.m. on Thursday. The results were shown in Table 6. It can be seen from the results that, after different administration routes of different samples, the effects on ovulation in mice were basically the same. Therefore, in the follow-up work, the mouse experiments were also performed by administeration intraperitoneally, while other animals were appropriately adjusted according to specific operations. Preferably, pigs, cattle, and goats were administered intramuscularly.

**Table 6 Effects of different administration routes on the number of ovulation mice (ovum/mouse)**

| Samples/ administration routes | Intraperitoneally | Subcutaneously | Intramuscularly |
|---|---|---|---|
| Recombinant 4D8 | 22.1±1.54 | 20.1±2.04 | 22.0±1.34 |
| Recombinant 1E5 | 24.2±2.03 | 22.6±1.93 | 23.9±2.16 |
| Ascites-derived 4D8 | 22.2±1.61 | 21.0±1.86 | 22.4±1.97 |
| Ascites-derived 1E5 | 23.9±1.95 | 21.3±1.90 | 24.0±1.57 |
| Polyclonal antibodies of goat | 12.3±2.32 | 11.1±1.96 | 11.8±2.33 |
| PMSG | At 10IU, the number of ovulation was 18.9±2.48 | | |
| Normal saline | 0 | | |

### Example 7: Application of monoclonal antibody in improving ovulation quality of ICR Mice

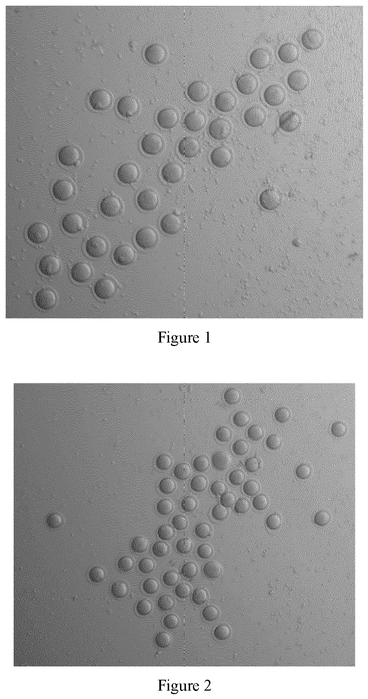

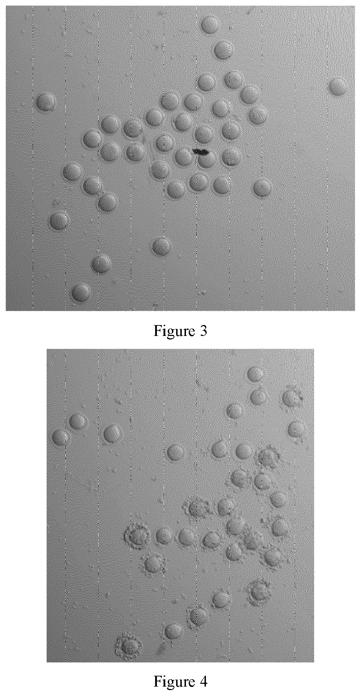

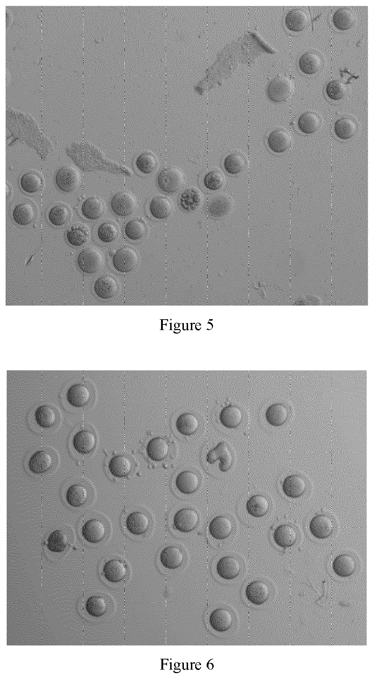

5-week-old female ICR mice were selected and randomly divided into 6 groups, 10 mice in each group cmprising: 1 µg/kg recombinant monoclonal antibody 4D8, 1 µg/kg recombinant monoclonal antibody 1E5, 1 µg/kg ascites-derived monoclonal antibody 4D8, 1 µg/kg ascites-derived monoclonal antibody 1E5, 10IU PMSG, and 1µg/kg goat polyclonal antibody. At 5:00 p.m. on Monday, the above dosages of antibodies and 10IU PMSG were injected intraperitoneally, and 10 IU HCG was also injected intraperitoneally 48h later. Oocytes were dissected at 9:00 a.m. on Thursday, and the quality of the eggs was observed. The results were shown in Figures 1-6. It can be seen that, the eggs in the antibody group were all in the M2 stage, the hyalin membrane was apparent, and there were no dead eggs. Similarly, it can be concluded that recombinant monoclonal antibodies and ascites-derived monoclonal antibodies have less difference on the effects on ovulation quality.

### Example 8: Effects of Anti-inhibin Monoclonal Antibody on Conception Rate and Litter Size in Mice

The *in vivo* activities of recombinant and ascites-derived monoclonal antibodies 4D8 and 1E5 were detected by comparing the conception rate and litter size of mice. The present disclosed products were intended to be used to replace PMSG in the field of animal reproduction, wherein PMSG was used as a standard, and normal saline as a negative control.

7-week-old female ICR mice were selected and randomly divided into 7 groups, 10 mice in each group. At 5:00 p.m. on the same day, 0.02 µg antibody and 10 IU PMSG were injected into each mouse intraperitoneally. 10 IU HCG were injected 48h later, and those mice were bred. After 20 days, the conception rate was counted, and the litter size was counted after calving.

The results were shown in Table 7: under the antibody dosage of 1 µg/kg, the conception rate (90%) of the monoclonal antibody groups was significantly higher than that of the PMSG group (80%), and also higher than that of the goat polyclonal antibody group (70%). The average litter size was at least 13, which was also higher than the 11.1 of PMSG and 10 of goat polyclonal antibodies. At the same time, it can be seen that both the recombinantly expressed monoclonal antibody and the ascites-derived monoclonal antibody performed well in terms of conception rate and average litter size, but the reason why the recombinant monoclonal antibodies was slightly superior may be that the mouse IgG contained in ascites caused negative effects on the experiments.

Preferably, application research was carried out subsequently with recombinant monoclonal antibody 4D8 and recombinant monoclonal antibody 1E5.

**Table 7 Effects of monoclonal antibodies 4D8 and 1E5 on conception and litter size in mice**

| Group | The number of mice | Conception mice | Conception rate | Litter size | Average litter size |
|---|---|---|---|---|---|
| Recombinant 4D8 | 10 | 9 | 90% | 123 | 13.7±0.93 |
| Recombinant 1E5 | 10 | 10 | 100% | 134 | 13.4±0.86 |
| Ascites-derived 4D8 | 10 | 9 | 90% | 118 | 13.1±1.00 |
| Ascites-derived 1E5 | 10 | 9 | 90% | 120 | 13.3±0.75 |
| PMSG | 10 | 8 | 80% | 89 | 11.1±0.89 |
| Goat polyclonal antibodies | 10 | 7 | 70% | 70 | 10.0±0.99 |
| Normal saline | 10 | 4 | 40% | 28 | 7.0±1.43 |

### Example 9: Application of different dosages of antibodies in increasing the litter size of gilts

110 of 210-day-old three-crossbred gilts having a weight of 85-100kg and similar physical signs were selected. Randomly divided into 11 groups: recombinant monoclonal antibody 4D8 (0.1 µg/kg, 1 µg/kg, 1,000 µg/kg), recombinant monoclonal antibody 1E5 (0.1 µg/kg, 1 µg/kg, 1,000 µg/kg), 10 IU PMSG, goat polyclonal antibody (0.1 µg/kg, 1 µg/kg, 1,000 µg/kg); normal saline was used as a negative control. The above-mentioned dosages of different medications were injected intramuscularly at the back and neck of donor gilts in each group, and 500 IU HCG was injected 80h later. The first insemination was performed 24h after administration of HCG, and the second insemination was performed at an interval of 16h.

The results were shown in Table 8: the total litter size of gilts in the 1µg/kg monoclonal antibody 4D8 group and 1E5 group (125 and 131 heads) was higher than that of the 10IU PMSG group (72 heads), and 1µg/kg and 1,000µg/kg goat polyclonal antibody group, and there was a significant difference when compared with the PMSG group (P<0.05). The average litter size (13.9 and 13.1) of the 1µg/kg monoclonal antibodies 4D8 and 1E5 groups was also higher than that of the 10IU PMSG group (10.3 head), and the 1µg/kg and 1,000 µg/kg goat polyclonal antibody groups (9.4 headss).

**Table 8 Comparison of recombinant monoclonal antibody 4D8, 1E5, PMSG, and goat polyclonal antibody on litter size in gilts**

| Group | Number of sows | Number of litter sows | Total litter size | Average litter size |
|---|---|---|---|---|
| Monoclonal antibody 4D8-L | 10 | 4 | 39 | 9.8±2.22 |
| Monoclonal antibody 4D8-M | 10 | 9 | 125 | 13.9±1.52 |
| Monoclonal antibody 4D8-H | 10 | 9 | 120 | 13.3±1.78 |
| Monoclonal antibody 1E5-L | 10 | 5 | 41 | 8.2±2.01 |
| Monoclonal antibody 1E5-M | 10 | 10 | 131 | 13.1±1.15 |
| Monoclonal antibody 1E5-H | 10 | 9 | 118 | 13.1±1.43 |
| PMSG | 10 | 7 | 72 | 10.3±1.24 |
| Goat polyclonal antibody-L | 10 | 2 | 15 | 7.5±2.31 |
| Goat polyclonal antibody -M | 10 | 7 | 66 | 9.4±1.39 |
| Goat polyclonal | 10 | 7 | 66 | 9.4±1.56 |
| antibody -H | | | | |
| Normal saline | 10 | 4 | 30 | 7.5±2.01 |

| | | | | |
|---|---|---|---|---|
| Note: L refers to 0.1µg/kg, M refers to 1µg/kg, L refers to 1,000 µg/kg | | | | |

### Example 10: Application of antibodies in promoting simultaneous estrus and ovulation in silts and sows

80 of three-crossbred gilts and 80 of sows without estrus at 2 week after weaning, with weight of 85-100kg, the same breed and similar signs, were selected. On the premise of no dosage optimization, the dosage for pigs was calculated based on the theoretical dosage and the results of *in vivo* activity in mice, and pigs were randomly divided into 4 groups: 1 µg/kg recombinant monoclonal antibody 4D8 group, 1 µg/kg recombinant monoclonal antibody 1E5 group, 10IU PMSG group, 1µg/kg goat polyclonal antibody group; each group was further divided into gilts and sows.

In each group of donor pigs, the above dosages of different medicines were intramuscularly injected at the back of the ear and neck, 500IU HCG were injected 80h later, and the estrus of the sows in each group was observed. After 72h, the eggs of donor pigs were surgically collected, and the number of ovulations was calculated.

In the prior art, the ovulation number of normal natural estrous sows is usually 8-14 pieces/sow (see Chinese invention patent application CN111134084A; King B, et al. Ovulatory and endocrine responses after active immunization of gilts against a synthetic fragment of bovine inhibin.Journal of animal science.1993;71(4):975-82;Ri-hong G, et. al., A novel method to improve sow reproductive performance combination of pre-weaning immunization against inhibin and post-insemination hCG treatment. Journal of Integrative Agriculture. 2020:0). As shown in Table 9, the donor pigs in the recombinant monoclonal antibody 4D8 group and the recombinant monoclonal antibody 1E5 group were in good estrus, and under conditions of administration in the dosage of 1 µg/kg, the estrus number of gilts and sows in the recombinant monoclonal antibody group was higher than 95%, which was higher than PMSG group (85% and 80%) and goat polyclonal antibody group (70% and 65%). And the ovulation number of sows in each group was higher than that of normal natural estrous sows (8-14 pieces/head); similarly, at the dosage of 1 µg/kg, the average number of ovulations per pig in gilts and sows of the recombinant monoclonal antibody 4D8 group was 24.8 and 25.5 respectively, and the average number of ovulations per pig in gilts and sows of the recombinant monoclonal antibody 1E5 group was 24.7 and 25.2, which were higher than those in the PMSG group (19.8 and 20.1), goat polyclonal antibody group (18.3 and 17.9). The difference between the two groups was significant compared with the PMSG group (P<0.05).

**Table 9 Effects of recombinant monoclonal antibody 4D8/1E5, PMSG, and goat polyclonal antibody on estrus synchronization and ovulation in gilts and sows**

| Group | Recombinant monoclonal antibody 4D8 | | Recombinant monoclonal antibody 1E5 | | PMSG | | Goat polyclonal antibody | |
|---|---|---|---|---|---|---|---|---|
| | Gilts | Sows | Gilts | Sows | Gilts | Sows | Gilts | Sows |
| Number of pigs | 20 | 20 | 20 | 20 | 20 | 20 | 20 | 20 |
| Number of estrous pigs | 20 | 19 | 20 | 20 | 17 | 16 | 14 | 13 |
| Estrus rate | 100% | 95% | 100% | 100% | 85% | 80% | 70% | 65% |
| Average ovulation rate | 24.8±2.05 | 25.5±2.13 | 24.7±1.99 | 25.2±2.33 | 19.8±1.76 | 20.1±2.13 | 18.3±2.01 | 17.9±2.34 |

### Example 11: Application of antibody in promoting superovulation in cows

Under natural conditions, a cow produces only one embryo at a time, which greatly limits the breeding of high-quality cattle, so it is necessary to develop a method of producing multiple high-quality embryos at a time.

110 heads of 3-6-year-old Holstein cows with physical health and no diseases were selected and randomly divided into 11 groups: recombinant monoclonal antibody 4D8 (0.1 µg/kg, 1 µg/kg, 1,000 µg/kg), recombinant monoclonal antibody 1E5 (0.1 µg/kg, 1 µg/kg, 1,000 µg/kg), 10IU PMSG, goat polyclonal antibody (0.1µg/kg, 1µg/kg, 1,000 1µg/kg); normal saline was used as a negative control. Cows in each group were fed with 1 kg of concentrate feed on the basis of the original feeding, and were given the corresponding medications in the above dosages by intramuscular injection according to conventional superovulation schemes, and the estrus was observed. The first insemination was performed at 12h after standing in estrus, and the second insemination was performed at 24h later. Embryos were collected by non-surgical washing on Day 16, and the number of embryos was counted.

The results were shown in Table 10: the number of embryos (8.1 and 8.4) per cow in the 1 µg/kg recombinant monoclonal antibody 4D8 group and recombinant monoclonal antibody 1E5 group was higher than that of the PMSG group (6.2), 1 µg/kg and 1,000 µg/kg goat polyclonal antibody group (5.1). The difference was significant (P<0.05). Similarly, the number of available embryos (6.5 and 7.0) per cow in the 1 µg/kg recombinant monoclonal antibody 4D8 group and recombinant monoclonal antibody 1E5 group were higher than those in the PMSG group (4.9), 1 µg/kg and 1,000 µg/kg goat polyclonal antibody group (3.5). The difference was significant (P<0.05).

**Table 10: Comparison of recombinant monoclonal antibody 4D8/1E5, PMSG and goat polyclonal antibody in promoting superovulation of Holstein cows**

| Group | Number of cows | Number of embryos per cow | Number of available embryos per cow |
|---|---|---|---|
| Monoclonal antibody 4D8-L | 10 | 3.4±1.21 | 2.7±0.81 |
| Monoclonal antibody 4D8-M | 10 | 8.1±0.59 | 6.5±0.55 |
| Monoclonal antibody 4D8-H | 10 | 8.0±0.78 | 6.6±0.21 |
| Monoclonal antibody 1E5-L | 10 | 3.6±1.13 | 2.8±0.54 |
| Monoclonal antibody 1E5-M | 10 | 8.4±0.41 | 7.0±0.49 |
| Monoclonal antibody 1E5-H | 10 | 8.3±0.39 | 7.0±0.31 |
| PMSG | 10 | 6.2±0.48 | 4.9±0.43 |
| Goat polyclonal antibody-L | 10 | 1.5±0.99 | 1.1±0.77 |
| Goat polyclonal antibody-M | 10 | 5.1±0.62 | 3.5±0.58 |
| Goat polyclonal antibody -H | 10 | 5.1±0.59 | 3.5±0.43 |
| Normal saline | 10 | 0.8±0.92 | |

| | | | |
|---|---|---|---|
| Note: L refers to 0.1µg/kg, M refers to 1µg/kg, H refers to 1,000µg/kg. | | | |

### Example 12: Application of antibodies in improving eestrous situation of anestrous cows

It can be seen from Example 11 that the dosage of 1 µg/kg had good results in superovulation of cows, and was more feasible in the actual application process. Therefore, it was planned to use this dosage to carry out research on improving the anestrus condition of cows .

After estrus detection, 40 of Holstein cows that could not be in normal estrus were selected and randomly divided into four groups: 1 µg/kg recombinant monoclonal antibody 4D8, 1 µg/kg recombinant monoclonal antibody 1E5, 10 IU PMSG and 1 µg/kg goat polyclonal antibody. The donor cows in each group were injected in the above-mentioned dosages of corresponding medications by intramuscular injection at fixed time points according to the estrus synchronization procedure, and the estrus was observed by bull climbing and rectal examination.

The results were shown in Table 11, the anestrous cows were sensitive to the medications. The estrus rate of cows in 1µg/kg recombinant monoclonal antibody 4D8 group and recombinant monoclonal antibody 1E5 group was 80%, higher than that in PMSG group (60%) and goat polyclonal antibody group (50%). The difference was significant (P<0.05).

**Table 11 Comparison of recombinant monoclonal antibody 4D8/1E5, PMSG, and goat polyclonal antibody on inducing estrus in anestrous cows**

| Group | Number of cows | Number of estrus cows | Estrus rate |
|---|---|---|---|
| Recombinant monoclonal antibody 4D8 | 10 | 8 | 80% |
| Recombinant monoclonal antibody 1E5 | 10 | 8 | 80% |
| PMSG | 10 | 6 | 60% |
| Goat polyclonal antibody | 10 | 5 | 50% |

### Example 13: Application of antibody in promoting estrus synchronization and promoting twinning rate in female goats.

110 of 1.5-3-year old female goats having a weight of 30-45kg, physical health and no disease were selected and randomly divided into four groups: recombinant monoclonal antibody 4D8 (0.1µg/kg, 1µg/kg, 1,000µg/kg), recombinant monoclonal antibody 1E5 (0.1µg/kg, 1µg/kg, 1.000µg/kg). 10IU PMSG, goat polyclonal antibody group (0.1µg/kg, 1µg/kg, 1,000µg/kg); normal saline was used as a negative control. On any day in the estrus cycle, a progesterone vaginal suppository was placed in the donor goat and recorded as Day 0. Each donor goat was injected intramuscularly with 300 IU of the corresponding drug (Day 10), and the suppository was withdrawn (Day 12). The estrous performance of the female goats was observed and tested the estrus using a test ram. The redness of genitalia, mucus flow and acceptance of climbing were regarded as estrus, and the estrus rate was calculated. At the same time, the first insemination was performed at 24h after estrus, and the second insemination was performed after an interval of 16h. The conception rate and twinning rate were counted.

The results were shown in Table 12, the female goats in each group were in estrus period obviously , and the estrus rate of female goats in the dosage of 1 µg/kg in recombinant monoclonal antibody 4D8 and recombinant monoclonal antibody 1E5 groups reached greater than 90%, which was higher than that in the 10IU PMSG group (70%) and goat polyclonal antibody group (30%), and there was a significant difference (P<0.05) compared with the PMSG group; similarly, the conception rates of female goats in the recombinant monoclonal antibody 4D8 and recombinant monoclonal antibody 1E5 groups reached greater than 90%, which was higher than that of the PMSG group (80%), 1 µg/kg and 1,000 µg/kg goat polyclonal antibody group (30%). It can be seen from the results of twins that, the twin pregnancy rate of the 1 µg/kg recombinant monoclonal antibody 4D8 and recombinant monoclonal antibody 1E5 group was higher than 60%, which was significantly higher than that of the PMSG group (43.8%) and the goat polyclonal antibody group (33.3%). The difference was significant (P<0.05).

**Table 12 Comparison of the effects of recombinant monoclonal antibody 4D8/1E5, PMSG, and goat polyclonal antibody on estrus and twin lambs of female goats**

| Group | Number of test goats | Number of estrus goats | Estrus rate | Number of conception goats | Conception rate | Twinning rate |
|---|---|---|---|---|---|---|
| Monoclonal antibody 4D8-L | 10 | 4 | 40% | 3 | 30 | 33.3% |
| Monoclonal antibody 4D8-M | 10 | 10 | 90% | 9 | 90% | 66.7% |
| Monoclonal antibody 4D8-H | 10 | 9 | 90% | 8 | 90% | 62.5% |
| Monoclonal antibody 1E5-L | 10 | 5 | 50% | 4 | 50% | 50.0% |
| Monoclonal antibody 1E5-M | 10 | 9 | 90% | 10 | 100% | 70% |
| Monoclonal antibody 1E5-H | 10 | 9 | 90% | 8 | 80% | 62.5% |
| PMSG | 10 | 7 | 70% | 7 | 70% | 43.8% |
| Goat polyclonal antiboy-L | 10 | 2 | 20% | 1 | 10% | 0% |
| Goat polyclonal antiboy -M | 10 | 4 | 40% | 3 | 30% | 33.3% |
| Goat polyclonal antiboy -H | 10 | 4 | 40% | 3 | 30% | 33.3% |
| Normal saline | 10 | 2 | 20% | 2 | 20% | 0% |

| | | | | | | |
|---|---|---|---|---|---|---|
| Note: L refers to 0.1µg/kg; M refers to 1µg/kg; H refers to 1,000µg/kg. | | | | | | |

## Claims

1. An anti-inhibin antibody or antigen-binding portion thereof, comprising a combination of CDRs selected from heavy chain and light chain below:
(1) heavy chain CDR1, CDR2 and CDR3 comprising the sequences of SEQ ID NO.1, 2, 3 or any variant thereof respectively, and light chain CDR1, CDR2 and CDR3 comprising the sequences of SEQ ID NO. 4, 5, 6 or any variant thereof respectively;
(2) heavy chain CDR1, CDR2 and CDR3 comprising the sequences of SEQ ID NO. 11, 12, 13 or any variant thereof respectively, and light chain CDR1, CDR2 and CDR3 comprising the sequences of SEQ ID NO.14, 15,16 or any variant thereof respectively;
preferably, the antibody or antigen-binding portion thereof comprises a heavy chain variable region having the amino acid sequence selected from SEQ ID NO. 7, 17 or any vaiant thereof, and a light chain variable region having the amino acid sequence selected from SEQ ID NO. 9, 19 or any variant thereof.

2. The anti-inhibin antibody or antigen-binding portion thereof of claim 1, comprising the heavy chain variable region having the amino acid sequence of SEQ ID NO. 7 or any variant thereof, and the light chain varible region having the amino acid sequence of SEQ ID NO.9 or any variant thereof.

3. The anti-inhibin antibody or antigen-binding portion thereof of claim 1, comprising the heavy chain variable region having the amino acid sequence of SEQ ID NO. 17 or any variant thereof, and the light chain varible region having the amino acid sequence of SEQ ID NO. 19 or any variant thereof.

4. Nucleic acid molecule encoding the anti-inhibin antibody or antigen-binding portion thereof of any of claims 1-3;
preferably, the nucleic acid molecule comprises a nucleic acid sequence selected from SEQ ID NO. 8, 18 or any variant thereof that encodes an antibody heavy chain variable region, and a nucleic acid sequence selected from SEQ ID NO. 10, 20 or any variant thereof that encodes an antibody light chain variable region;
more preferably, the nucleic acid molecule comprises a nucleic acid sequence shown in SEQ ID NO. 8 or any variant thereof that encodes a heavy chain variable region, and a nucleic acid sequence shown in SEQ ID NO. 10 or any variant thereof that encodes a light chain variable region.
more preferably, the nucleic acid molecule comprises a nucleic acid sequence shown in SEQ ID NO. 18 or any variant thereof that encodes a heavy chain variable region, and a nucleic acid sequence shown in SEQ ID NO. 20 or any variant thereof that encodes a light chain variable region.

5. A vector comprising the nucleic acid molecule of claim 4.

6. A cell comprising the nucleic acid of claim 4 or the vector of claim 5.

7. An expression system, which is constructed by introducing an expression vector comprising the nucleic acid of claim 4 into a host cell;
the expression vector comprises any one selected from bacterial plasmids, yeast plasmids, plant cell viruses and mammalian cell viruses (such as adenovirus or retrovirus); more preferably, the expression vector is pcDNA3.1(+ );
the host cell is a prokaryotic host cell or a eukaryotic host cell;
preferably, the prokaryotic host cell is a bacterial cell; preferably, the bacterial cell is *Escherichia coli;*
preferably, the eukaryotic host cell is selected from any one of fungi, plants, insects and mammals;
ppreferably, the fungal eukaryotic host cell is selected from any one of yeast (such as *Pichia pastoris* and *Saccharomyces cerevisiae*) and filamentous fungi;
preferably, the mammalian eukaryotic host cell is selected from any one of Chinese hamster ovary (CHO) cells, murine myeloma (NSO) cells, baby hamster kidney (BHK) cells and human embryonic kidney (HEK) cells (such as HEK293 cells); more preferably, the host cell is the Chinese hamster ovary (CHO) cells.

8. The expression system of claim 7, wherein the method of introducing the expression vector into the host cell is selected from transfection, transformation or infection;
preferably, the expression vector is introduced into the host cell by transfection;
preferably, the transfection includes: electroporation transfection, calcium phosphate transfection, liposome transfection, protoplast fusion transfection, microinjection, electroporation, gene gun, cationic polymer and viral vector infection.

9. A composition comprising the antibody or antigen-binding portion thereof of any one of claims 1-3, the nucleic acid of claim 4, the vector of claim 5, and/or the cell of claim 6.

10. Use of the anti-inhibin antibody or antigen-binding portion thereof of any one of claims 1-3, the nucleic acid of claim 4, the vector of claim 5 and/or the cell of claim 6 and/or the composition of claim 9 in the preparation of medications for promoting animal reproduction, estrus synchronization, conception and birth, embryo transfer, improving ovulation quality, promoting livestock reproduction, or inducing estrus in female livestock;
preferably, the animals are thereof selected from mice, pigs, cattle, goat.
